Europäisches Patentamt

European Patent Office

Office européen des brevets

(11) Publication number: **0 362 879 B1**

# EUROPEAN PATENT SPECIFICATION

(45) Date of publication of patent specification: **17.08.94**  (51) Int. Cl.5: **A61K 31/74**, A61K 47/34

(21) Application number: **89118605.8**

(22) Date of filing: **06.10.89**

(54) **Pyrogen-adsorbing polymers.**

(30) Priority: **07.10.88 JP 254342/88**

(43) Date of publication of application:
**11.04.90 Bulletin 90/15**

(45) Publication of the grant of the patent:
**17.08.94 Bulletin 94/33**

(84) Designated Contracting States:
**BE CH DE FR GB LI SE**

(56) References cited:
**GB-A- 2 092 470**
**US-A- 4 491 660**

**CHEMICAL ABSTRACTS, vol. 101, no. 24, 10th December 1984, page 297, abstract no. 216189v, Columbus, Ohio, US;**

**PATENT ABSTRACTS OF JAPAN, vol. 13, no. 372 (C-627)[3720], 17th August 1989;**

**Heitz W. and Winau H., "Gelchromatographie", Die Macromolekulare Chemie, 131, (1970), 75-88;**

**Moore J.C. "Gel Permeation Chromatography "I. A new method ....." J. of Polymer Science, A 2, (1964), 835-843**

(73) Proprietor: **Hirayama, Chuichi**
**No. 373-12, Shimonabe-machi**
**Kumamoto-shi Kumamoto-ken (JP)**

Proprietor: **Ihara, Hirotaka**
**3-21-9, Takahira**
**Kumamoto-shi Kumamoto-ken (JP)**

Proprietor: **Ajinomoto Co., Inc.**
**No. 15-1, Kyobashi 1-chome**
**Chuo-ku**
**Tokyo (JP)**

(72) Inventor: **Hirayama, Chuichi**
**373-12 Shimonabe-machi**
**Kumamoto-shi Kumamoto-ken (JP)**
Inventor: **Ihara, Hirotaka**
**854-2 Takahira**
**Shimizu-machi**
**Kumamoto-shi Kumamoto-ken (JP)**
Inventor: **Tsunoda, Shunsei**
**2136-58 Nirenoki**
**Shimizu-machi**
**Kumamoto-shi Kumamoto-ken (JP)**
Inventor: **Aihara, Katsutoshi**
**970-8 Iwano**
**Ueki-machi**
**Kamoto-gun Kumamoto-ken (JP)**

Inventor: **Yagyu, Kazufumi c/o Central Research Lab.**
**Ajinomoto Co. Inc**
**1-1 Suzuki-cho**
**Kawasaki-ku**
**Kawasaki-shi Kanagawa-ken (JP)**
Inventor: **Honma, Masao c/o Central Research Lab.**
**Ajinomoto Co. Inc**
**1-1 Suzuki-cho**
**Kawasaki-ku**
**Kawasaki-shi Kanagawa-ken (JP)**


(74) Representative: **Strehl Schübel-Hopf Groening & Partner**
**Maximilianstrasse 54**
**D-80538 München (DE)**

## EP 0 362 879 B1

**Description**

This invention relates to a method for removing pyrogens by the use of a water-insoluble pyrogen adsorbent which is effective in purging medicines, tonics, etc. of pyrogens, i.e., substances which, on entering living bodies in consequence of intravenous administration, e.g., by injection of medicines, tonics, produce fever.

In the production of medicines , for intravenous administration, the technique of purging medicines , of pyrogens has a profound significance.

The term "pyrogens" as used herein refers to substances classified as exotoxins which are excreted by gram-positive bacteria, e.g. Corynebacterium diphtheriae, Staphylococcus aureus, and endotoxins which are components of cellular walls of such gram-negative bacteria such as Escherichia coli. Of these pyrogens, generally those of the latter case, i.e., the endotoxins occurring in the gram-negative bacteria pose a problem. They have been identified as originating in lipid A, a glycolipid constituting itself the active center in the liposaccharide (LPS), i.e., the composite of a lipid with polysaccharide.

It is assumed that when a medicine entraining a pyrogen is intravenously administered, e.g., by injection, the pyrogen produces fever by acting on heat centers such as the hypothalamus. The fever may be very serious and sometimes may bring about death from shock. A manufacturer engaged in the production of a medicine for injection, therefore, is obliged to conduct tests with test animals and confirm safety of the medicine by establishing that the medicine does not entrain any pyrogens.

Among existing methods available for the removal of such pyrogens from medicines, the method which effects the removal by adsorption with carbon powder or ion-exchange resin, the method which attains the removal by decomposing the pyrogen with an acid or an alkali, and the method which accomplishes the removal in a selective manner by the use of an ultra-membrane filter, for example, are renowned. A case or the successful application of the composite having an imidazole-containing compound such as histamine or a nucleic acid base bonded to a dextran type gel carrier for the adsorptive removal of pyrogens has been reported in literature [Minobe et al: Journal of Chromatography, 262, 193-198 (1983)]. With respect to the conventional methods of the removal of pyrogens from medicines during their production, however, there remain many technical problems to be solved. The medicines themselves to be subjected to the treatment are not stable and the amounts of pyrogens to be removed are extremely small as compared with the amounts of medicines under treatment. With these and other drawbacks as contributory factors, the methods mentioned above have many technical problems yet to be solved for the purpose of effective application for the removal of pyrogens in the production of medicines on a commercial scale. It has been recognized in this field, that it is desirable to develop an effective and expeditious method capable of eliminating all of these problems.

Under these circumstances, it has been found that a novel pyrogen adsorbent comprising beads consisting of a polymer of amino acids as a carrier and having an imidazole derivative deposited fast on the carrier excels in affinity for pyrogens and enjoys advantages in pyrogen adsorption treatment owing to the rigidity of the carrier (Japanese Patent Application Laid-Open (Kokai) HEI 1-127,039 (1989)). The preparation of the pyrogen adsorbent, however, requires a process of several reaction steps and inevitably entails more or less operational intricacies. Moreover, the pyrogen adsorbent has a limited capacity for the incorporation of the imidazole derivative as a ligand and, consequently, a limited capacity for the adsorption of pyrogens.

According to another method for removing pyrogens (GB-A-2 092 470) a pyrogen-containing solution is contacted with an adsorbent which comprises a water-insoluble carrier and a nitrogen-containing heterocyclic compound which may contain a functional group, e.g. an amino, carboxy or hydroxy group. According to this reference the heterocyclic compound is bonded to the carrier either directly or through a spacer. US-A-4 491 660 describes an adsorbent for endotoxins which comprises a matrix material of the formula A - x - B - y - Z, wherein A is an insoluble polymer, x is a first linking group comprising isourea, ester, ether or amine, B is a spacer group comprising a straight, branched or cyclic alkyl from 1 to 12 carbon atoms, hydroxyl, loweralkylamine, loweralkylether or loweralkylthioether, y is a second linking group comprising a methylene, ether, thioether or amide group and Z is an aryl nucleus group which may be substituted.

The abstract of JP 59-112 888 in Chemical Abstracts, vol. 101, 1984, page 297 relates to the removal of gram-negative bacteria and cell wall material from water by a treatment with fibers containing amino groups. As a specific example of such fibers there are stated (chloroacetamido)methylated polystyrene fibers.

GB-A-2 092 470 discloses a method for reducing the pyrogen content of or removing pyrogens from solutions contaminated therewith. The respective solution is contacted with an adsorbent, which comprises a water-insoluble carrier and a nitrogen-containing heterocyclic compound. It is reported that the adsorbents

3

may be packed in a column and the solution passed therethrough. The trapped pyrogen may then be washed from the column which is reusable.

US-A-4 491 660 describes an endotoxin binding material fixed to a support. Said material is reported to be capable of binding endotoxin and comprises polar, water-insoluble, high molecular weight polymer supports to which are attached groups which adsorb said endotoxin molecules. The groups comprise bifunctional aliphatic molecules which are on one end bound to the support, and on the other hand bound to an aryl group.

JP-A-59-112888 describes the removal of bacteria from water contaminated with gram-negative bacteria. The water is contacted with fibers containing amino groups.

Considering the above state of the art it is therefore an object of this invention, to provide a method for removing pyrogens by means of a pyrogen adsorbent having excellent ability for the selective adsorption and being applicable to the production of medicines on a commercial level.

In view of the problems mentioned above, the inventors have further continued a diligent study to find that a water-insoluble substance which is formed of a polymer possessing amide groups and which may optionally contain solely a modifying group having an aliphatic group and/or an aryl group in the side chain and/or at the terminal of the main chain thereof exhibits extremely high affinity for pyrogens and possesses a remarkable ability to effect adsorptive removal of pyrogens from medicinal substances, etc., intended for intravenous administration e.g., by injection. This invention has been accomplished as the result.

The present invention provides a method for removing pyrogens from a pyrogen containing solution by contacting the solution with a water insoluble pyrogen adsorbent which is composed of a polymer possessing amide groups selected from the group consisting of:

(1) a polymer of amino acids,
(2) a derivative of a polymer of amino acids obtainable by esterification of free carboxy groups to form hydrophobic ester groups, by acylating free amino groups, by reacting free amino groups with an isocyanate compound or by introducing a basic functional group by the reaction with an alkyl amine or an amine linked to aromatic compounds through a spacer, wherein derivates obtained by modifying polyglutamic acids having the side chains amidated with ethylendiamine, with glutaric acid, are excluded,
(3) a nylon,
(4) a polyacrylamide.

Various methods have, as has been described, been tried for the removal of pyrogens. In the operation for the removal of pyrogens from medicines in the production of pharmaceuticals, the selective removal of pyrogens by adsorption is frequently not attained fully as expected because of the problem that the amount of pyrogens to be removed is very small as compared with the relatively high concentration of the medicinal substance in the material under treatment. Thus, it has been recognized that it is necessary to develop an absorbent possessing highly selective affinity for pyrogens and to find a method of removing pyrogens in high efficiency. It is further required that the method for the removal of pyrogens as a step in the pharmaceutical production allows a quick treatment in addition to having selective affinity.

The pyrogen adsorbent used according to this invention exhibits prominent affinity for pyrogens owing to the use of an amide group-containing polymer in place of agarose gel or dextran type sepharose gel which has found utility in the conventional technique for adsorbents. Further, the pyrogen adsorbent used in this invention possesses an ability to effect efficient and quick separation and removal of pyrogens from a medicinal substance under treatment for purification owing to the characteristic of its high rigidity as compared with the rigidity of the conventional polysaccharide gel.

The aliphatic group-containing modifying groups which are usable for the pyrogen adsorbent of this invention include alkyl groups represented by methyl group and cyclohexyl group, allyl groups such as vinyl group, ether groups such as methoxy group, carboxyl group, ester groups represented by ethoxycarbonyl group, acyl groups such as acetyl group and cyclohexylcarbonyl group, alkylamino groups such as methylamino group and aminoethylamino group, carbamoyl group, and mesyl group, for example.

The aryl group-containing modifying groups include aryl groups such as phenyl group, aralkyl groups such as benzyl group, ether groups such as phenoxy group and benzyloxy group, ester groups represented by benzyloxycarbonyl group, acyl groups such as benzoyl group, amino groups such as benzylamino group which are linked to aromatic compounds through an aliphatic spacer, and toluenesulfonyl group, for example.

The amide group-containing polymers which are usable as the pyrogen adsorbent of the present invention include poly-amides which are polymers of amino acids, nylons composed of diamines and dibasic acids, and polyacrylamides which are polymers of acrylic acid amides, for example.

More specifically, the poly-amides include inherently hydrophobic amino acid polymers composed of neutral amino acids such as alanine, phenylalanine, valine, and leucine, for example.

4

In addition, hydrophilic poly amino acid polymers which have been rendered hydrophobic (i.e. hydrophilic amino acid polymers having incorporated therein a hydrophobic group) are also usable and within the concept of "amide group - containing polymer". The hydrophilic poly amide acid derivatives which comply with the foregoing description include those obtained by hydrophobically esterifying with hydrophobic ester groups the free carboxyl groups of acidic poly amide acids such as poly glutamic acids and poly aspartic acids. Specifically, examples are represented by alkyl esters, benzyl esters, dicyclohexanemethyl esters, and tetrahydropyran methanol esters of such poly amino acids. Besides, those obtained by protecting basic amino acids such as lysine and ornithine with a hydrophobic group, specifically represented by carbobenzoxylated products and carboethoxylated produces of such amino acids, are other examples, and are within the concept of "amide group - containing polymer".

The amino acid polymer to be used in the present invention may be either a homopolymer of one amino acid or a copolymer of two or more amino acids.

Those obtained by acylating the terminal amino group of the amino acid polymers mentioned above, specifically represented by acetylated products and benzoylated products of amino acid polymers are other examples and are within the concept of "amide group - containing polymer". Those obtained by the reaction of amino acid polymers with an isocyanate compound, specifically represented by the incorporation of carbamide in amino acid polymers are further examples within that concept.

Copolymers of a amino acid polymer or a polymer of an amino acid derivative with a urethane prepolymer or an epoxy resin are also usable and are within that concept. polymers of amino acid derivatives which are not suitable for the pyrogen adsorbent of this invention include those glutamic acid derivative polymers obtained by modifying with glutaric acid such polymer of glutamic acids having the side chains thereof amidated with ethylene diamine.

The pyrogen adsorbent may acquire enhanced adsorptive activity by incorporating a basic functional group into the amino acid polymers or their derivatives. The polymers of amino acid derivatives rendered hydrophobic, e.g., by esterification, specifically represented by poly glutamic acid methyl ester, acquire a still higher ability of pyrogen adsorption by amination of their ester moiety, and the hydrophobic derivatives of lysine, ornithine etc., inherently having an amino group in the side chains thereof, specifically represented by poly(N$^{\epsilon}$-carbobenzoxylysine), may acquire an enhanced ability for pyrogen adsorption by eliminating the hydrophobic group in the manner conventionally practised in the art.

The term "basic functional group" as used herein refers preferably to an amino group, especially an aliphatic amino group In greater detail, alkyl amines represented by ethyl amine, aliphatic amines such as ethylene diamine, diethylene diamine, and benzyl amine, and amines linked to aromatic compounds through a spacer are usable and within the purviews of this invention. Substantially all of the basic functional groups which are usable in the present invention possess acid ionization indexes, pKa, of not less than 6.5. The incorporation of these basic functional groups into the aforementioned amino acid polymer or its derivative is accomplished either directly or indirectly through a spacer.

By effecting the incorporation through a spacer, the adsorbent used according to this invention can be provided with outstanding affinity for pyrogens. The spacer thus used must be selected so as to suit the amino acid polymer or its derivative of which the adsorbent is to be formed. In the case of a polymer of amino acid derivatives, including a glutamic acid derivative or an aspartic acid derivative, for example, a polyamine having two to six carbon atoms is advantageously used as the spacer.

For the incorporation under discussion, the number of basic functional groups to be incorporated is not critical.

The amino acid polymers or their derivatives to be used for the pyrogen adsorbent of the present invention are not specifically limited. However, in consideration of the ease with which the raw material is procured, i.e., the ease of synthesis, and the ease of chemical modification to be made subsequently to the preparation of adsorbent beads, it is desirable to use, e.g., a poly glutamic acid ester, a poly aspartic acid ester, or a hetero-polymer partly composed of a glutamic acid ester or an aspartic acid ester and mainly of other hydrophobic amino acid, or polylysine or polyornithine both rendered hydrophobic, or a hetero-polymer partly composed of hydrophobic polylysine or hydrophobic polyornithine and mainly of other hydrophobic amino acid. In the preparation of the adsorbent of this invention which is composed of a glutamic acid derivative and/or an aspartic acid derivative, for example, the amino acid derivative polymer aimed at can be produced by using a methyl ester, an ethyl ester, a propyl ester, or a benzyl ester of the relevant amino acid as the raw material.

Though the polymerization degree of the amino acid polymer or its derivative is not specifically restricted, it is desired to exceed 100, preferably to fall in the range of 100 to 1,000, from the viewpoint of the strength of the pyrogen adsorbent.

The nylons which are usable in the present invention include 6-nylon, 6,6-nylon, 6,10-nylon, 6,12-nylon, 11-nylon, and 12-nylon, for example. In consideration of the ease of procurement of raw material and the ease of synthesis, it is advantageous to use 6-nylon, 6,6-nylon, or 12-nylon. A mixture of two or more of these nylon species or a copolymer thereof may be used.

The preparation of the nylons can be easily accomplished by thermal polymerization or interfacial polycondensation as practised in the art.

The polyacrylamide derivatives include a polymer which is obtained by copolymerizing acrylamide or methacrylamide with N,N'-methylenebisacrylamide The resultant copolymer is insoluble in water. From the viewpoint of the strength, the content of N,N'-methylenebisacrylamide in this polymer is desired to exceed 50%.

The preparation of the polyacrylamide derivative is easily carried out by photopolymerization or radical polymerization as practised in the art.

The shape of the pyrogen adsorbent of this invention is not specifically restricted. The adsorbent may be spherical particles, fibers, film, or powder, for example.

Particularly, the spherical particles, i.e. one of the forms in which the pyrogen adsorbent used according to this invention is put to use, are made of a polymer of amino acids (synthetic polyamino acid) or a nylon, which is totally different material from the polysaccharide type materials used in the conventional adsorbent in the form of spherical particles. The present inventors have already invented basic spherical particles of such a polymer of amino acids as described above and a process for the production thereof, and filed a patent application on these inventions (EP-A-211223)

In the preparation of the spherical particles of the polymers of amino acids used as the pyrogen adsorbent in the present method, these spherical particles can be obtained by using an inherently hydrophobic polymer of amino acid or a polymer of amino acid derivative rendered hydrophobic as a raw material.

To be specific, the spherical particles are formed by dissolving either a hydrophobic polymer of amino acids or a polymer of amino acid derivatives rendered hydrophobic in an organic solvent, adding the resultant solution to an aqueous medium (in which said resultant solution does not dissolve or is sparingly soluble), causing the added solution to be suspended in the aqueous medium, and expelling the organic solvent from the resultant suspension. The spherical particles may be further modified as occasion demands.

Only so long as the spherical particles mentioned above are allowed to assume the form of a hydrophobic polymer of amino acid derivative particularly during the step for preparation of a dispersion, the spherical particles of a hydrophobic polymer of amino acid derivatives and a hydrophilic polymer of amino acid derivatives inclusive of an amphipatic polymer of amino acid derivatives can be finally obtained by eliminating the protective group after the formation of spherical particles.

The selection from among the hydrophobic and the hydrophilic particles consisting of a polymer of amino acid derivatives to be prepared depends heavily on the quality of the medicinal substance, etc. from which pyrogens are to be removed. Generally when a medicinal substance to be treated for purification is hydrophilic, for example, both the hydrophilic and hydrophobic particles consisting of a polymer of amino acid derivatives can be invariably effectively used without any particular restriction. On the other hand, when a medicinal substance happens to be hydrophobic, however, the hydrophilic particles consisting of a polymer of amino acid derivatives employed more advantageously.

Among organic solvents to be used in the preparation of the spherical particles consisting of a polymer of amino acids as the pyrogen adsorbent of this invention, those which dissolve the hydrophobic amino acid polymer or its derivative satisfactorily, exhibit no solubility in water, and possess a lower boiling point than an aqueous medium solvent, are most preferred. The organic solvents which prove to be advantageous for use herein include chloroform, dichloromethane, dichloroethane, other similar halogenated hydrocarbons, benzene, and mixtures thereof. In the preparation of this nature, the solution obtained by polymerizing an amino acid or an amino acid derivative in an organic solvent thereby producing a hydrophobic amino acid polymer or a polymer of an amino acid derivative can be used in its unmodified form.

The diameter of the spherical particles can be easily controlled by the viscosity of the organic solvent-aqueous medium system and the stirring speed. Generally, the diameter of the produced spherical particles increases proportionally when the concentration of the amino acid polymer or the polymer of an amino acid derivative in the organic solvent increases and the viscosity of the aqueous medium system decreases. The spherical particles can be obtained with a small diameter by increasing the stirring speed. The control of the diameter of the spherical particles can be further facilitated by the addition of a viscosity regulator such as partially acetified polyvinyl alcohol or gelatin.

The particle diameter is not critical, but usually selected from the range of 5 to 300 $\mu$m

The spherical particles can be easily prepared with a porous texture. Specifically, this preparation is accomplished by following the procedure of the preparation of the spherical particles described above, except that an additive exhibiting incompatibility with the hydrophobic amino acid polymer or the polymer of amino acid derivatives rendered hydrophobic and compatibility with the organic solvent having dissolved the polyamino acid or polyamino acid derivative and possessing a higher boiling point than the organic solvent or an aqueous medium is added to the solution of the hydrophobic polymer of amino acids or the polymer of amino acid derivatives rendered hydrophobic in the organic solvent. The additives which answer this description include decalin, tetralin, toluene, xylene, ethylbenzene, diethylbenzene, anisole, hexanol, octanol, dibutyl ether, cyclohexane, paraffins, phthalic acid esters such as dibutyl phthalate, higher fatty acid esters such as methyl dodecanoate, and higher saturated fatty acids such as oleic acid, for example. By selecting the kind of the additive and adjusting the amount of the additive to be used, the porous spherical particles can be obtained with a pore diameter in the range of $10^2$ to $10^7$ as expressed in the molecular weight of water-soluble polysaccharide and a porosity in the range of 10 to 99%. To be particularly desirable for this invention, the spherical particles possess a porosity in the range of $10^3$ to $10^7$.

When these spherical particles are prepared by the method described above, they can be produced with a freely controlled diameter. Generally, the spherical particles obtained by this method acquire a diameter in the range of 1 to 300 $\mu$m. However, by adjusting the viscosity of the reaction solution or the stirring speed, the diameter of the spherical particles can be adjusted in the range of 0.1 to 1 mm. The particle size is selected so as to suit the particular use to which the produced spherical particles are put. When the spherical particles are intended for use in column chromatography, for example, the spherical particles having a diameter in the range of 5 to 300 $\mu$m prove to be advantageous.

The spherical particles of the quality described above are characterized by the fact that they are rigid as compared with the polysaccharide type spherical particles in popular use to date. An analysis of the infrared absorption spectrum of these spherical particles revealed the presence of the $\beta$-structure at least partly. It is believed that the presence of the $\beta$-structure contributes in a large measure to the rigidity of the spherical particles.

The preparation of the pyrogen adsorbent in the form of fibers is carried out easily by the conventional method. Specifically, the fibers of pyrogen adsorbent are obtained by preparing a solution of a polymer of amino acids or a polymer of amino acid derivatives in a organic solvent and extruding this solution into an organic solvent which is incompatible with the polymer of amino acids or the polymer of amino acid derivatives. The diameter of these fibers is suitably selected by the diameter of the spinning nozzle to be used for the spinning. Generally, the fibers 30 to 60 $\mu$m in diameter are actually put to use.

The fibers of a nylon are prepared by melt spinning.

The diameter of the fibers is not critical, but usually in the range of 30 - 60 $\mu$m.

The preparation of the pyrogen adsorbent in the form of film can be easily carried out by the conventional method. Specifically, the film of pyrogen adsorbent can be obtained, e.g., by preparing a solution of a polymer of amino acids or a polymer of amino acid derivatives in an organic solvent, casting the solution on a glass plate, and expelling the organic solvent from the cast sheet of the solution. The thickness of this film is suitably selected at the time that the solution is cast on the glass sheet. The nylon film or the film of a polyacrylamide derivative is prepared by the method of melt extrusion.

The thickness of films is not critical, but usually in the range of 10 - 50 $\mu$m.

The preparation of the pyrogen adsorbent in the forms of powder is carried out by any of the various methods described herein below, for example. The powder of a polymer of amino acids or a polymer of amino acid derivatives can be easily prepared by heterogeneous polymerization. Specifically, the powder can be obtained by polymerizing an amino acid or amino acid derivative in an organic solvent compatible with an amino acid N-carboxy anhydride, a polymerizable monomer, and incompatible with the polymer of amino acids to be subsequently produced thereby inducing precipitation of a corresponding polymer of amino acids or a polymer of amino acid derivatives, and separating the precipitate. The preparation of the nylon powder is effected by the method which comprises melting the nylon at an elevated temperature in an organic solvent compatible with the nylon at the elevated temperature and incompatible with the nylon at normal room temperature and cooling the resultant hot solution thereby allowing the suspended nylon particles to settle or by the method of mechanical pulverization. The powder of a polyacrylamide derivative can be easily obtained by photopolymerization.

The size of powder is not critical, but usually in the range of 5 - 300 $\mu$m.

The product obtained by treating the surface of a porous carrier of silica or alumina with a polymer of amino acids or a polymer of amino acid derivatives, nylon, or a polyacrylamide derivative can be used. The porous carrier having the surface thereof treated with a polymer of amino acids or a polymer of amino acid derivatives can be easily obtained by dispersing a porous carrier in the solution of the polymer of amino acid or the polymer of amino acid derivatives in an organic solvent, then separating the impregnated carrier from the dispersion, and drying the separated carrier. The porous carrier to be used in this case is not specifically restricted.

An inorganic carrier of silica or alumina which has a large specific surface area can be advantageously used. The fibers formed of a material such as cellulose, Polyester, polyacrylonitrile, a polyolefin etc., and having the surface thereof coated with a polymer of amino acids or a polymer of amino acid derivatives are also usable. Though the fibers thus used have no particular restriction, the ease with which they are put to use increases in proportion as their specific surface area increases.

The porous carrier and the fibers which have their surfaces coated with a polymer of amino acids or a polymer of amino acid derivatives, nylon, or a polyacrylamide derivative are characterized by the fact that, similarly to the spherical particles, they possess high rigidity and a large specific surface area as compared with the conventional spherical particles of polysaccharide type. Thus, they are capable of efficiently and quickly removing pyrogens from a finally refined medicinal substance. Further, they are economically advantageous over the spherical particles in the sense that they need the polymer in a small application ratio.

The removal of pyrogens by the use of the pyrogen adsorbent described above can be executed by either column chromatography or batchwise treatment. In the method of column chromatography, a medicinal substance freed of pyrogens can be obtained according to this invention by filling a column with the pyrogen adsorbent, washing the packed column with a suitable buffer, then passing a pyrogen-containing solution of the medicinal substance through the packed column, and collecting the fraction flowing out of the column. In the method of batchwise treatment, the medicinal substance freed of pyrogens can be obtained by stirring the pyrogen adsorbent in a pyrogen-containing solution of the medicinal substance and then removing the adsorbent from the resultant mixture.

The pyrogen adsorbent used in the present invention exhibits virtually no swelling property and allows the treatment of column chromatography to be quickly carried out thereon as compared with the conventional adsorbent particles of agarose or dextrin. Thus, it functions optimally when it is used as a quick pyrogen-removing agent in the commercial production of a medicinal substance.

The pyrogen adsorbent used in this invention possesses excellent affinity for pyrogens and, at the same time, abounds in rigidity and consequently in stability and exhibits an extremely small swelling degree as compared with the conventional adsorbent particles using an agarose or dextran type substance and, therefore, allows the operation for removal of pyrogens to be carried out quickly thereon.

Moreover, the pyrogen adsorbent used according to this invention can be prepared very simply and conveniently without involving any special reaction path. Further, since this pyrogen adsorbent possesses low affinity for the medicinal substance subjected to purification, it is capable of removing pyrogens with extremely high selectivity without a sacrifice of the recovery rate of the finally refined medicinal substance.

The feature of the pyrogen adsorbent used according to this invention, i.e. the optimum function the adsorbent fulfils in the adsorption of pyrogens as demonstrated above, may be safely ascribed to the synergism between the effect the amide group-containing polymer's structure produces on the mechanism of adsorption of pyrogens and the affinity the introduced basic functional group, particularly the weakly basic amino group, manifests for the pyrogens. It is also noteworthy that in the introduction of the basic functional group, the particular kind of the group can be suitably selected and the ratio of the introduction can be easily controlled.

EXAMPLES

Now, the present invention will be described more specifically below with reference to working examples.

Preparation of pyrogen adsorbent

Example 1:

A solution of 10 g of poly methyl-L-glutamate and 10 ml of decalin in 400 ml of dichloroethane was added dropwise to 2,000 ml of an aqueous 1.5 w/v% partially acetylized polyvinyl alcohol kept at 50°C. When the resultant mixture was vigorously stirred at the same temperature for 24 hours, dichloroethane was expelled by vaporization and spherical particles of decalin-containing poly methyl-L-glutamate were obtained.

The spherical particles were washed to be freed of decalin by the method of Soxhlet extraction using acetone, suspended in water, and passed through standard sieves of Japanese Industrial Standard (JIS) to obtain spherical particles of diameters in the range of 64 to 105 $\mu$m. These spherical particles had the maximum pore diameter of 2 x 10$^4$ as expressed in the molecular weight of water-soluble polysaccharide and a porosity of 55%.

The particles thus obtained were labeled as "Adsorbent A (PG-OMe)".

Example 2:

In a mixed solution consisting of 50 ml of methanol and 50 ml of ethylene diamine, 5 g of Absorbent A obtained in Example 1 was suspended and then stirred gently at 65°C for 48 hours. The particles were collected by filtration and then washed with methanol and water, to obtain amino group-introduced particles.

The amount of amino groups introduced in the particles was determined by the ion electrode method to be 3.2 meq/g (3.2 milliequivalents per 1 g of the particles).

The particles thus obtained were labeled as "Adsorbent B (PG-NH$_2$)".

Comparative Experiment 1:

In 100 ml of tetrahydrofuran, 4 g of Adsorbent B obtained in Example 2 was suspended and 0.14 g of glutaric anhydride was added thereto. The resultant mixture was gently stirred at 40°C for 24 hours. The particles which were consequently produced in the mixture were collected by filtration and then washed with methanol and water, to obtain carboxyl group-introduced particles. By the method of titration, it was confirmed that not less than 98% of the amino groups in the particles had been carboxylated.

The particles thus obtained were labeled as "Adsorbent C (PG-COOH)".

Comparative Experiment 2:

In 50 ml of water, 3.5 g of Adsorbent C obtained in Comparative Experiment 1 and 3 g of histamine dihydrochloride were suspended and then slowly stirred in conjunction with 4.5 ml of triethyl amine and 0.56 g of a water-soluble carbodiimide [such as, for example, 1-cyclohexyl-3-(2-morpholinoethyl)-carbodiimide-p-toluenesulfonate] at room temperature for two days. The particles consequently formed in the stirred mixture were collected by filtration and then washed sequentially with water, alcohol, and ether.

By the method of amino acid analysis, the histamine content of the particles was found to be 0.2 meq/g.

The particles thus obtained were labeled as "Adsorbent D (PG-His)".

Example 3:

At room temperature, 10 g of poly-L-leucine and 10 ml of diethylbenzene and 500 ml of benzene were stirred for several hours to swell the polymer and then were heated to 70°C, to give rise to a viscous transparent solution. This solution was added dropwise to 2,000 ml of an aqueous 2.5 w/v% partially acetylized polyvinyl alcohol solution. The resultant mixture was vigorously stirred for 24 hours with the temperature of the system gradually being lowered to 40°C. Consequently, benzene was expelled by vaporization and spherical particles of diethylbenzene-containing polyleucine were obtained.

The spherical particles were washed to be freed of diethyl-benzene by the method of Soxhlet extraction using acetone and then passed through standard sieves of JIS. Consequently, there were obtained spherical particles of diameters in the range of 10 to 25 $\mu$m.

The particles thus obtained were labeled as "Adsorbent E (PLeu)".

Example 4:

A solution of 10 g of poly(N$^\epsilon$-carbobenzoxy-L-lysine) in 10 ml of diethylbenzene and 400 ml of chloroform was added dropwise to 2,000 ml of an aqueous 1.5 w/v% partially acetylized polyvinyl alcohol solution kept at 40°C. When the resultant mixture was vigorously stirred at the same temperature for 24 hours, the chloroform was expelled by vaporization and spherical particles of diethylbenzene-containing poly(N$^\epsilon$-carbobenzoxy-L-lysine) were obtained.

The spherical particles were washed to be freed of diethylbenzene by the method of Soxhlet extraction using acetone and then passed through standard sieves of JIS, to produce spherical particles of diameters in the range of 64 to 105 $\mu$m. To 10 g of the spherical particles were added 30 ml of 25% hydrogen bromide/acetic acid and 300 ml of ether, and the mixture was stirred at room temperature for one hour, there were obtained poly-L-lysine particles partially freed of carbobenzoxy groups.

The particles thus obtained were labeled as "Adsorbent F (PLys)".

Example 5:

A solution of 1.0 g of L-leucine-N-carboxylic anhydride and 0.2 g of L-isoleucine-N-carboxylic anhydride in 15 ml of acetonitrile was heated to 40°C. The solution, on addition thereto of 4 $\mu$l of triethyl amine, began to form a powdery polymer.

On elapse of six hours thereafter, the system was cooled to room temperature to induce precipitation of the powdery polymer. The polymer was separated by filtration and dried. Consequently, 0.9 g of a copolymer of L-leucine and L-isoleucine was obtained.

The particles thus obtained were labeled as "Adsorbent G (PLeu-Ile)".

Example 6:

A 10 wt% solution poly methyl-L-glutamate in dichloroethane was heated to a temperature in the range of 40° to 50°C and deaerated under vacuum.

The spinning dope consequently obtained was placed in a spinning syringe and discharged from the syringe at a fixed rate into kerosene. Fibers were obtained by drawing the poly methyl-L-glutamate coagulated at the leading end part of the nozzle of the syringe into a coagulation bath and taking up the coagulated thread on a bobbin kept in rotation at a fixed rate.

The fiber thus obtained was labeled as "Adsorbent H (Fibrous PG-OME)".

Example 7:

A solution of 1.4 g of L-glutamic acid methyl-N-carboxy anhydride in 10 ml of ethyl acetate was heated to 40°C. The solution, on addition thereto of 5 $\mu$l of triethyl amine, began to educe poly methyl-L-glutamate. On elapse of four hours thereater, the system was cooled to room temperature to induce precipitation of the poly methyl-L-glutamate. The polymer was separated by filtration and then dried. Consequently, there was obtained 1 g of poly methyl-L-glutamate.

The particles thus obtained were labeled as "Adsorbent I (Powder PG-OME)."

Example 8:

A solution of 1 g of poly methyl-L-glutamate in 100 ml of dichloroethane was stirred gently with 1 g of porous silica (particle diameters of 0.5 to 11 $\mu$m) at room temperature for two hours. The resultant mixture was deaerated under vacuum for 5 minutes, to displace the interior of the porous silica with the polymer solution. The procedure just described was repeated twice. Then, the porous silica was separated by filtration, washed with methanol, and then dried. Consequently, there was obtained 1 g of porous silica having the surface thereof treated with poly methyl-L-glutamate.

The particles thus obtained were labeled as "Adsorbent J (surface-treated PG-OME)".

Example 9:

In a mixed solvent consisting of 4 ml of water and 1 ml of ethanol, 0.5 g of acrylamide and 0.5 g of N,N'-methylenebisacrylamide were dissolved. To the resultant solution, 10 mg of benzophenone-2,4'-dicarboxylic acid was added. The solution thus obtained was deaerated under vacuum for about 15 minutes and then spread in a thickness of 1 to 2 mm on the bottom of a petri dish.

The layer of the solution, on exposure to the ultraviolet light from a high-pressure mercury-vapor lamp for 5 minutes, turned into a white solid film. This solid film was removed from the petri dish and crushed. Consequently, 0.9 g of polyacrylamide was obtained. The particles thus obtained were labeled as "Adsorbent K (Paa)".

Adsorptive removal of pyrogens

Example 10:

Samples, 0.1 to 0.2 g (dry weight), of the adsorbents prepared in Examples 1 to 4 and Comparative Experiments 1 and 2 and two commercially available adsorbents (produced by A company and B company) were each washed sequentially with a 10 mM pyrogen-free phosphate buffer of pH 7.5 (containing 1.0 M NaCl) and a 10 mM pyrogen-free phosphate buffer of pH 7.5 (containing 0.07 M NaCl). The respective washed samples were brought into contact with 1 to 2 ml of various bacterial toxoid solutions containing pyrogens. The eluates or supernatants consequently formed were tested for pyrogen content.

The ratio of adsorption was found by subtracting the amount of pyrogen remaining in the eluates or supernatants from the amount of pyrogen contained initially in the bacterial toxoid solutions, dividing the difference by the amount of pyrogen contained initially in the bacterial toxoid solutions, and multiplying the resultant quotient by 100. The results were as shown in Table 1.

From the results, it is clearly noted that Adsorbent A itself possessed a fairly distinct ability to adsorb pyrogens and Adsorbent B having weakly basic amino groups introduced into the side chain thereof through the medium of a spacer exhibited a further enhanced ability to adsorb pyrogens.

In contrast, Adsorbent C having carboxyl groups introduced in the side chain thereof showed virtually no ability to adsorb pyrogens, indicating that the carboxyl groups suppressed the ability to adsorb pyrogens.

Adsorbent D having imidazole derivatives (histidine) introduced in the side chain of adsorbent C exhibited a pyrogen-adsorbing ability equivalent to the ability of the Adsorbent A but not comparable with the ability of Adsorbent B. When Adsorbent A using chitosan as the main body of adsorbent and Adsorbent B using sepharose as the carrier and having imidazole linked thereto through the medium of a spacer were used, their effects determined by the method employed herein were not insignificant but were not fully satisfactory as expected.

Adsorbent E using polyleucine, a hydrophobic poly amino acid, as the main body of adsorbent and Adsorbent F inherently possessing amino groups in the side chain thereof each showed an appreciable pyrogen-adsorbing ability, supporting the practical utility of the present invention.

Table 1  Adsorbent's capacity for adsorptive removal of pyrogens

| Pyrogen adsorbent | (g) | Ratio of adsorption (%) | | |
|---|---|---|---|---|
| | | Concentrated crude toxoid A (originating in Chlostridium titani); pyrogen concentration 3,933 ng/ml | Concentrated crude toxoid B (originating in Chlostridium titani); pyrogen concentration 1,080 ng/ml | Concentrated crude toxoid C (originating in Diphtheria); pyrogen concentration 397 ng/ml |
| **This invention** | | | | |
| Adsorbent A ($PG-OMe$) | 0.194 | 81 | 86 | 68 |
| Adsorbent B ($PG-NH_2$) | 0.185 | 95 | 100 | 100 |
| Adsorbent E (PLeu) | 0.156 | 78 | 83 | 86 |
| Adsorbent F (PLys) | 0.168 | 86 | 92 | 98 |
| **Comparison** | | | | |
| Adsorbent C ($PG-COOH$) | 0.115 | 0 | 0 | – |
| Adsorbent D ($PG-His$) | 0.101 | 0 | 16 | 91 |
| Adsorbent L | 0.200 | 0 | 0 | 72 |
| Adsorbent M | 0.124 | 20 | 4 | 54 |

Example 11:

A mixture of 0.05 g (dry weight) of Adsorbent A prepared in Example 1 and 0.05 g (dry weight) of Adsorbent E prepared in Example 3 was treated by following the procedure of Example 10 and tested for the residual pyrogen content. The ratio of adsorption was found in the same manner as in Example 10.

As the result, the ratio of adsorption of pyrogen was found to be 81%. This value was practically equal to the values found for Adsorbents A and B under the conditions mentioned above.

Example 12:

Samples, 0.1 g (dry weight), of the adsorbents prepared in Examples 5 to 8 were each washed sequentially with a 10 mM pyrogen-free phosphate buffer of pH 7.5 (containing 1.0 M NaCl) and a 10 mM pyrogen-free phosphate buffer of pH 7.5 (containing 0.07 M NaCl).

The washed samples were each brought into contact with 1 to 2 ml of a phosphate buffer (pH 7.2) having 200 to 250 ng of pyrogens originating in varying bacteria. The eluates or supernatants consequently formed were tested for the residual pyrogen content.

The ratio of adsorption was determined in the same manner as in Example 10.

The results are as shown in Table 2. These adsorbents invariably exhibited highly satisfactory pyrogen-adsorbing activity.

Table 2

| Adsorbents and ratios of adsorption of pyrogen | |
| --- | --- |
| Pyrogen adsorbent | Ratio of adsorption (%) |
| Adsorbent G (P Leu-Ile) | 98 |
| Adsorbent H (Fibrous PG-OME) | 80 |
| Adsorbent I (Powdery PG-OME) | 85 |
| Adsorbent J (Surface-treated PG-OME) | 85 |

Example 13:

Samples, 0.05 g (dry weight), of 6-nylon particles (mean diameter 5 $\mu$m) and 12-nylon particles (mean diameter 5 $\mu$m) both available in the market, and the adsorbent prepared in Example 9 were washed sequentially with a 10 mM pyrogen-free phosphate buffer of pH 7.5 (containing 1.0 M NaCl) and a 10 mM pyrogen-free phosphate buffer of pH 7.5 (containng 0.07 M of NaCl).

These samples were each brought into contact with 1 to 2 ml of a phosphate buffer (pH 7.2) having 5 mg of bovine serum albumin dissolved therein. The supernatants consequently formed were tested for the residual pyrogen content. The ratio of adsorption was found in the same manner as in Example 10.

The results were as shown in Table 3. These adsorbents invariably showed highly satisfactory pyrogen-adsorbing activity. The ratios of recovery of bovine serum albumin were in the range of 75 to 90%.

Table 3

| Adsorbents and ratios of adsorption of pyrogen | |
| --- | --- |
| Pyrogen adsorbent | Ratio of as adsorption (%) |
| 6-Nylon | 85 |
| 12-Nylon | 95 |
| Adsorbent K (Paa) | 85 |

Example 14:

A mixture of 0.025 g (dry weight) of 6-nylon particles (mean diameter 5 $\mu$m) and 0.025 g (dry weight) of 12-nylon particles (mean diameter 5 $\mu$m) both available in the market was treated in the same manner as in Example 13 and tested for the amount of pyrogens. The ratio of adsorption was found in the same manner as in Example 10.

13

As the result, the ratio of adsorption of pyrogens was found to be 88%.

The detection of pyrogens was accomplished by the use of a toxinometer (produced by Wako Pure Chemical Industries Ltd.).

It is noted from the foregoing, particularly from the examples, that the pyrogen adsorbents of the present invention were invariably effective in removing pyrogens with very high selectivity without lowering the ratio of recovery of the medicinal substance subjected to purification.

The adsorbents were prepared very easily. Thus, they constitute themselves highly satisfactory adsorbents applicable for commercial production of pharmaceutical goods.

## Claims

1. A method for removing pyrogens from a pyrogen containing solution by contacting the solution with a water insoluble pyrogen adsorbent which is composed of a polymer possessing amide groups selected from the group consisting of:
   (1) a polymer of amino acids,
   (2) a derivative of a polymer of amino acids obtainable by esterification of free carboxy groups to form hydrophobic ester groups, by acylating free amino groups, by reacting free amino groups with an isocyanate compound or by introducing a basic functional group by the reaction with an alkyl amine or an amine linked to an aromatic compound through a spacer, wherein derivates obtained by modifying poly-glutamic acids having the side chains amidated with ethylendiamine, with glutaric acid, are excluded,
   (3) a nylon,
   (4) a polyacrylamide.

2. The method according to Claim 1, wherein the polymer of amino acids comprises a homopolymer of an amino acid or a copolymer of at least two amino acids.

3. The method according to Claim 1 or 2, wherein the polymer possessing amide groups is selected from the group consisting of copolymers of a polymer of amino acids or a polymer of amino acid derivatives with an urethane prepolymer or an epoxy resin.

## Patentansprüche

1. Verfahren zur Entfernung von Pyrogenen aus einer Pyrogen enthaltenden Lösung durch Inkontaktbringen der Lösung mit einem wasserunlöslichen Pyrogen-Adsorbens, das aus einem Amidgruppen enthaltenden Polymer gebildet ist, welches aus der Gruppe ausgewählt ist, die besteht aus :
   (1) einem Polymer aus Aminosäuren,
   (2) einem Derivat eines Polymers aus Aminosäuren, das erhältlich ist durch Verestern freier Carboxygruppen unter Bildung hydrophober Estergruppen, durch Acylieren freier Aminogruppen, durch Umsetzen freier Aminogruppen mit einer Isocyanatverbindung oder durch Einführen einer basischen funktionellen Gruppe durch die Reaktion mit einem Alkylamin oder einem durch einen Spacer an eine aromatische Verbindung gebundenen Amin, wobei Derivate ausgeschlossen sind, die durch Modifizieren von Polyglutaminsäuren, deren Seitenketten mit Ethylendiamin amidiert wurden, erhalten wurden,
   (3) einem Nylon,
   (4) einem Polyacrylamid.

2. Verfahren nach Anspruch 1, worin das Polymer aus Aminosäuren ein Homopolymer einer Aminosäure oder ein Copolymer von mindestens zwei Aminosäuren umfaßt.

3. Verfahren nach Anspruch 1 oder 2, worin das Amidgruppen aufweisende Polymer ausgewählt wird aus der Gruppe bestehend aus Copolymeren eines Polymers aus Aminosäuren oder eines Polymers aus Aminosäurederivaten mit einem Urethanpräpolymer oder einem Epoxyharz.

## Revendications

1. Un procédé pour éliminer les pyrogènes d'une solution contenant des pyrogènes par mise en contact de la solution avec un adsorbant de pyrogènes insoluble dans l'eau qui est composé d'un polymère possédant des groupes amides choisi dans le groupe constitué des suivants :

(1) un polymère d'aminoacides,

(2) un dérivé d'un polymère d'aminoacides pouvant être obtenu par estérification des groupes carboxy libres pour former des groupes esters hydrophobes, par acylation des groupes amino libres, par réaction des groupes amino libres avec un composé isocyanate ou par introduction d'un groupe fonctionnel basique par la réaction avec une alkylamine ou une amine liée à un composé aromatique par l'intermédiaire d'un espaceur, où les dérivés obtenus par modification des acides polyglutamiques ayant les chaînes latérales amidées par l'éthylène-diamine, par l'acide glutarique sont exclus,

(3) un Nylon,

(4) un polyacrylamide.

2. Le procédé selon la revendication 1, selon laquelle le polymère d'aminoacides comprend un homopolymère d'un aminoacide ou un copolymère d'au moins deux aminoacides.

3. Le procédé selon la revendication 1 ou 2, selon laquelle le polymère possédant des groupes amides est choisi dans le groupe constitué de copolymères d'un polymère d'aminoacides ou d'un polymère de dérivés d'aminoacides avec un prépolymère d'uréthanne ou une résine époxy.